# EUROPEAN PATENT APPLICATION

(11) **EP 3 120 792 A1**
(43) Date of publication of application: **25.01.2017**
(21) Application number: 15177832.1
(22) Date of filing: 22.07.2015
(51) Int. Cl.: A61B 18/02, A61B 17/00, A61B 18/00

(54) **CATHETER FOR CRYOGENIC ABLATION**

(71) Applicant: Gaita, Fiorenzo, 14010 Asti (IT)
(72) Inventor: Gaita, Fiorenzo, 14010 Asti (IT)
(74) Representative: Marchitelli, Mauro

(57) **Abstract**

Catheter for cryogenic ablation, particularly for treating atrial fibrillation, comprising: a grip (12), a hollow catheter body (14) a mapping catheter lumen (28) extending within the catheter body (14) and axially movable relative to the catheter body (14),a tube for injecting cooling fluid (42) that extends between the mapping catheter lumen (28) and the catheter body (14) a mapping catheter (48) movable within the mapping catheter lumen (28) having an end loop portion (50) perpendicular to the longitudinal axis (A) of the catheter body (14), a cryogenic balloon (34) having a first end (36) fixed to the distal end of the mapping catheter lumen (28) and a second end (38) fixed to the distal end (18) of the catheter body (14), characterized in that the distal end (32) of said mapping catheter lumen (28) has an open and tip-free front surface (56), and in that said first end (56) of said cryogenic balloon (34) is folded-back towards the inside of said cryogenic chamber (40), so that in the inflated position, said cryogenic balloon (34) has a front ablation area (58) that extends beyond the front surface (56) of said mapping catheter lumen (28).

## Description

### Field of the invention

The present invention refers to devices for cryogenic ablation and relates in particular to a balloon catheter particularly intended to be used for treating atrial fibrillation.

### Background of the invention

Atrial fibrillation is the most common cause of irregular heartbeat, with a prevalence of 1-2% of the general population, which progressively increases with age up to almost 10%. Depending on the persistence of the altered heartbeat, atrial fibrillation is defined as paroxysmal (recurrent episodes that terminate spontaneously within seven days), persistent (lasting more than seven days) or long-standing (lasting more than one year). The critical point in the majority of atrial fibrillation cases is the left atrium of the heart.

The therapeutic approach for treating atrial fibrillation varies according to the clinical form. For the paroxysmal form, the therapeutic objective is to insulate electrically the four pulmonary veins of the left atrium. For the persistent form, the therapeutic approach is much debated. Some authors argue that isolation of the pulmonary veins is sufficient for persistent forms while other authors consider it necessary to create additional lesions within the left atrium. For the long-standing form, there is a consensus on the fact that additional lesions on the left atrium wall are necessary to create lines for blocking activation of the electric front and/or to remove areas of abnormal conduction of electrical impulses (fragmented electrical potentials).

Currently, the treatment of atrial fibrillation is carried out with a transvenous percutaneous approach, minimally invasive for the patient. The most widely used treatment method for treating atrial fibrillation involves the use of radiofrequency intravenous catheters. These catheters allow tissue ablation to be performed in all clinical types of atrial fibrillation. However, these catheters have severe disadvantages in that the radiofrequency causes destruction of the architecture of the biological tissue, causing a coagulative necrosis of the tissues, which can result in thrombus formation and the risk of embolic events. Furthermore, because of the damage which is caused to the tissue, there is a risk of perforation of the wall subjected to the treatment.

For these reasons, alternative methods to radiofrequency ablation have been developed. Of particular relevance is cryogenic ablation. This treatment method carries out direct freezing of the tissue. The osmotic damage to the cells resulting from the cooling of the interstitium creates an electrically inert scar that keeps the tissue architecture intact and, therefore, reduces the risk of thrombi and perforation.

The current systems of cryogenic ablation for treating atrial fibrillation are based on the use of cryogenic balloon catheters. Examples of catheters for cryoablation are described in the documents US-A-2009/0182319, US-A-2010/0211056, and US-A-2013/0197497.

The catheters described in these documents comprise a grip, a hollow catheter body that extends from the grip, a mapping catheter lumen, which extends within the catheter body, a tube for injecting cooling fluid that extends between the mapping catheter lumen and the catheter body, and a cryogenic balloon having a first end fixed to a distal end of the catheter body and a second end fixed to a distal end of the mapping catheter lumen. In the catheters of known type, the distal end of the mapping catheter lumen has a rounded tip, which serves to facilitate cannulation of the pulmonary veins and the subsequent positioning of the cryogenic balloon at the level of the ostium of the pulmonary vein. The document US-A-2013/197497 also describes a mapping catheter extending within the mapping catheter lumen and having a loop portion located outside the mapping catheter lumen, which allows recording of the electrical potentials of the walls to guide the catheter and to verify the effect of the ablation.

The catheters described in these documents enable the effective implementation of cryogenic ablation of the ostium of the pulmonary veins. However, the catheters described in these documents are unsuitable for performing cryogenic ablation of the atrium walls. In fact, the tip of the mapping catheter lumen impedes the positioning of the cryogenic balloon against a flat surface, such as the left atrium wall. In principle, it would be possible to rest the side portion of the cryogenic balloon against a flat surface. However, the contact surface between the side wall of the cryogenic balloon and the atrium wall would be very limited. Furthermore, the flow of surrounding blood would disperse the cryogenic effect into the bloodstream rather than on the tissue to be treated. Therefore, these known catheters can be used for treating paroxysmal atrial fibrillation, but are unsuitable for treating persistent and long-standing atrial fibrillation, which requires ablation of portions of the left atrium wall.

The document US-A-2011/0184400 describes a catheter devoid of a central mapping catheter lumen and carrying a cryogenic balloon at its distal end. A solution of this type allows cryogenic ablation treatments on flat tissues and would therefore be potentially useful for treating persistent and long-standing forms of atrial fibrillation. However, the lack of the central mapping catheter lumen or of an internal guide for stabilizing the shape of the cryogenic balloon impedes adhesion of the cryogenic balloon to a circular ostium. The lack of a slot or a guide that facilitates cannulation of a blood vessel prevents the use of this device for treating the ostium of the pulmonary veins. Furthermore, this solution does not allow the use of a mapping catheter and does not allow the recording of electric potentials of the atrial wall, which is a procedure required to guide the ablation.

The document WO2006/009589 describes a cryoablation catheter having a cylindrical sleeve-shaped balloon with a flat front wall. The solution described in this document was developed for cryoablation of the pulmonary veins. The shape of the balloon may also allow cryoablation on a flat surface. This system is composed of two parallel catheters, an inner one, which terminates with a cryogenic tip and an outer one composed of the annular balloon filled with a saline solution, which is indirectly cooled by the inner catheter. The presence of a double system with an interface consisting of a balloon filled with a saline solution, which separates the cryogenic source from the target tissue of the ablation, results in a significant dispersion of the energy and a strong limitation of the heat exchange and the efficiency of the ablation. The cylindrical shape of the balloon does not allow positioning at the ostium of vessels of different diameters (the pulmonary veins can have widely varying diameters) nor the ability to completely occlude the incoming flow from the blood vessel. Furthermore, this system does not include an inner lumen for introducing a mapping catheter able to record electrical potentials within a pulmonary vein or fragmented electrical potentials on the free wall of the left atrium. For these reasons, a catheter of the type described in this document would not be suitable for use in clinical practice.

### Object and summary of the invention

The object of the present invention is to provide a catheter for cryogenic ablation which solves the problems of the prior art. In particular, the object of the invention is to provide a catheter capable of performing cryoablation both at the ostium of the pulmonary veins and on the flat walls of the atrium outside the pulmonary veins, and that has the possibility of inserting a mapping catheter inside it for guiding the ablation.

According to the present invention, this object is achieved by a catheter having the characteristics forming the subject of claim 1.

The claims form an integral part of the disclosure provided in relation to the invention.

### Brief description of the drawings

The present invention will now be described in detail with reference to the accompanying drawings, provided purely by way of non-limiting example, in which:
- Figure 1 is a perspective view of a catheter according to the present invention,
- Figure 2 is a cross-section on an enlarged scale according to the line II-II of Figure 1,
- Figure 3 is a cross-section corresponding to Figure 2 with the mapping catheter in the retracted position,
- Figure 4 is a cross-section corresponding to Figure 2 with the balloon in the relaxed position, and
- Figures 5-8 are schematic views illustrating the operation of the catheter according to the present invention.

### Detailed description

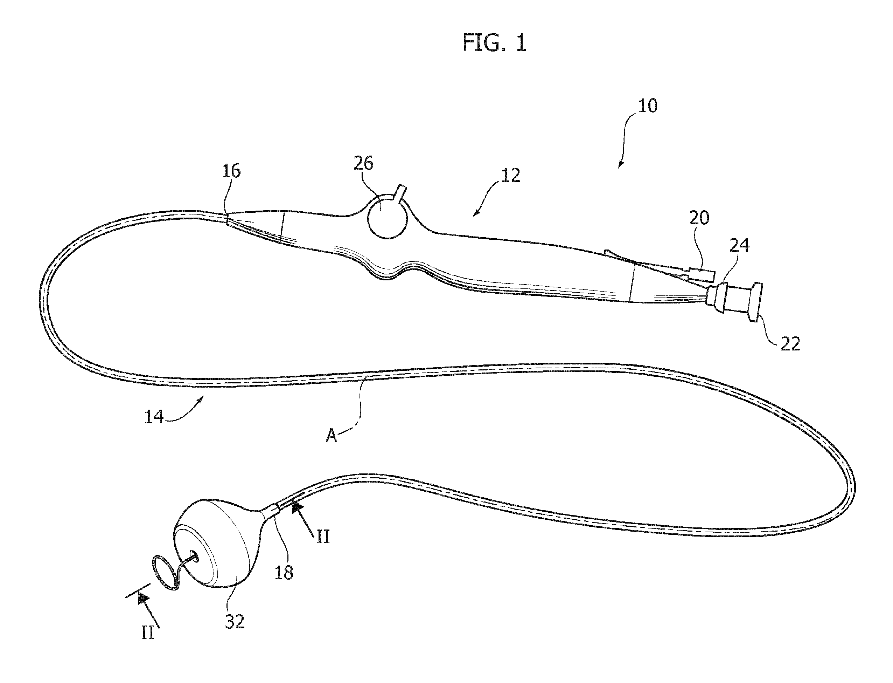

With reference to Figure 1, numeral 10 indicates a catheter for cryogenic ablation, in particular for treating atrial fibrillation. The catheter 10 comprises a grip 12 and a catheter body 14. The catheter body 14 is a hollow and flexible tubular element, which extends along its longitudinal axis A from a proximal end 16, connected to the grip 12, to a distal end 18. The grip 12 is of the type known per se in the field of intravenous catheters for cryoablation. The grip 12 comprises a port 20 for communicating with the inside of the catheter body 14. The grip 12 comprises a fluid connector 22 for connecting the catheter 10 to a source of cryogenic fluid. The grip 12 may also include a knob 26 for controlling the catheter body 14 according to modalities known per se. The constructive details of the grip 12 are not described in detail since they fall outside the scope of this invention. For a description of the structure and operation of the grip 12, reference can be made, for example, to the document US-A-2010/0211056.

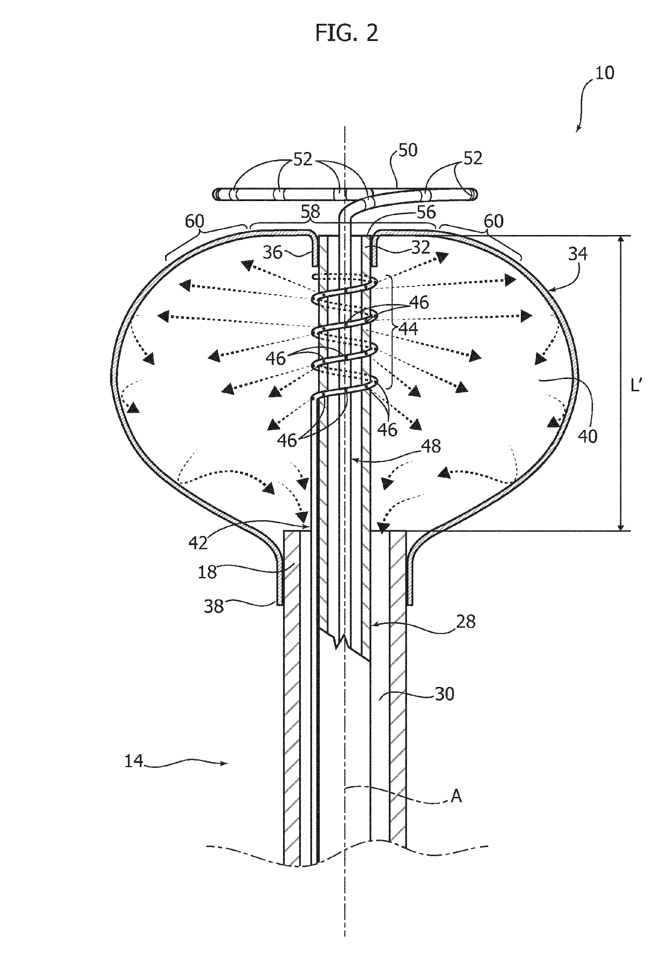

With reference to Figures 2, 3 and 4, the catheter 10 comprises a mapping catheter lumen 28, which extends within the catheter body 14. The mapping catheter lumen 28 has an outer diameter less than the inner diameter of the catheter body 14, so that an annular cavity 30 is defined between the catheter body 14 and the mapping catheter lumen 28. The mapping catheter lumen 28 consist of a flexible tube and is movable, relative to the catheter body 14, in the direction of the longitudinal axis A. The mapping catheter lumen 28 has a distal end 32, which extends beyond the distal end 18 of the catheter body 14.

The catheter 10 comprises a cryogenic balloon 34 having a first end 36 fixed to the distal end 32 of the mapping catheter lumen 28 and a second end 38 fixed to the distal end 18 of the catheter body 14. The cryogenic balloon 34 defines a cryogenic chamber 40 that surrounds the portion of the mapping catheter lumen 28 outside the catheter body 14. The cryogenic balloon 34 has a flexible wall, which is preferably formed by an inner membrane and an outer membrane superposed on each other. The outer membrane constitutes a safety layer that prevents dispersions of cryogenic gas in case of leakage of the inner membrane. In the drawings, the wall of the cryogenic balloon 34 is represented as if it were formed of a single layer, solely for reasons of simplicity of representation.

The catheter 10 comprises a tube for injecting cooling fluid 42, which extends into the annular cavity 30 defined between the mapping catheter lumen 28 and the catheter body 14. The tube for injecting cooling fluid 42 is parallel to the mapping catheter lumen 28 and has an injection zone 44 helically wound around the mapping catheter lumen 28 at the distal end 32 of the mapping catheter lumen 28. The injection zone 44 is provided with injection holes 46 through which the cryogenic fluid is injected into the cryogenic chamber 40. The cryogenic fluid injected through the injection holes 46 expands in the cryogenic chamber 40 and cools the cryogenic balloon 34. The cryogenic fluid keeps the cryogenic balloon 34 inflated and comes out of the cryogenic chamber 40 through the annular cavity 30.

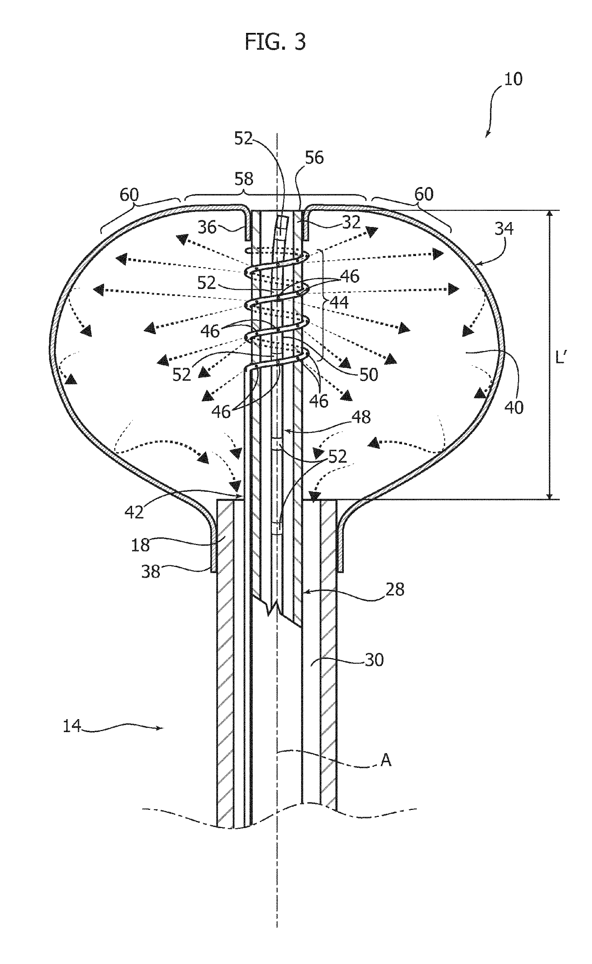

The catheter 10 comprises a mapping catheter 48, which extends within the mapping catheter lumen 28. The mapping catheter 48 is movable within the mapping catheter lumen 28 between an extracted position illustrated in Figure 2 and a retracted position illustrated in Figure 3. In the extracted position, the mapping catheter 48 has an end loop portion 50 perpendicular to the longitudinal axis A of the catheter body 14, and located on the outside of the mapping catheter lumen 28. In the retracted position, the end loop portion 50 assumes a straight shape and is pulled inside the mapping catheter lumen 28. The loop portion 50 of the mapping catheter 48 has an elastic memory whereby it assumes the loop shape when it is outside the mapping catheter lumen 28, while in the retracted position, it is retained in an extended position by the inner wall of the mapping catheter lumen 28. The end loop portion 50 is provided with electrodes 52 suitable for detecting electrical characteristics of the tissues. The mapping catheter 48 is able to carry out a mapping of the tissues to be treated and to provide information that confirms the cryogenic ablation treatment has taken place. A more detailed description of the structure and operation of the mapping catheter 48 is contained in the document US-A-2013/0197497.

The catheter 10 may be equipped with a temperature sensor (not illustrated) located at the distal end of the mapping catheter lumen 28 within the cryogenic balloon 34.

According to the present invention, the distal end 32 of the mapping catheter lumen 28 has an open and tip-free front surface 56. The distal end 32 of the mapping catheter lumen 28 is therefore devoid of a rounded tip that is present in the solutions according to the prior art for guiding the insertion of the catheter.

The first end 36 of the cryogenic balloon 34 is folded back towards the inside of the cryogenic chamber 40 and is fixed directly in contact with the outer surface of the distal end 32 of the mapping catheter lumen 28. The fixing between the first end 36 of the cryogenic balloon 34 and the distal end 32 of the mapping catheter lumen 28 can be carried out by gluing or by welding. The fixing of the first end 36 occurs on the outer surface of the cryogenic balloon 34 which, by effect of the folding-back, faces the outer side surface of the distal end 32 of the mapping catheter lumen 28. The second end 38 of the cryogenic balloon 34 is fixed on the outer surface of the catheter body 14. In this case as well, fixing can be carried out by means of gluing or welding. The fixing of the second end 38 occurs on the inner surface of the cryogenic balloon 34 which is facing and in contact with the outer side surface of the catheter body 14. In the case in which the wall of the cryogenic balloon 34 is formed by an outer membrane and an inner membrane, the outer surface of the outer membrane is fixed to the distal end 32 of the mapping catheter lumen 28.

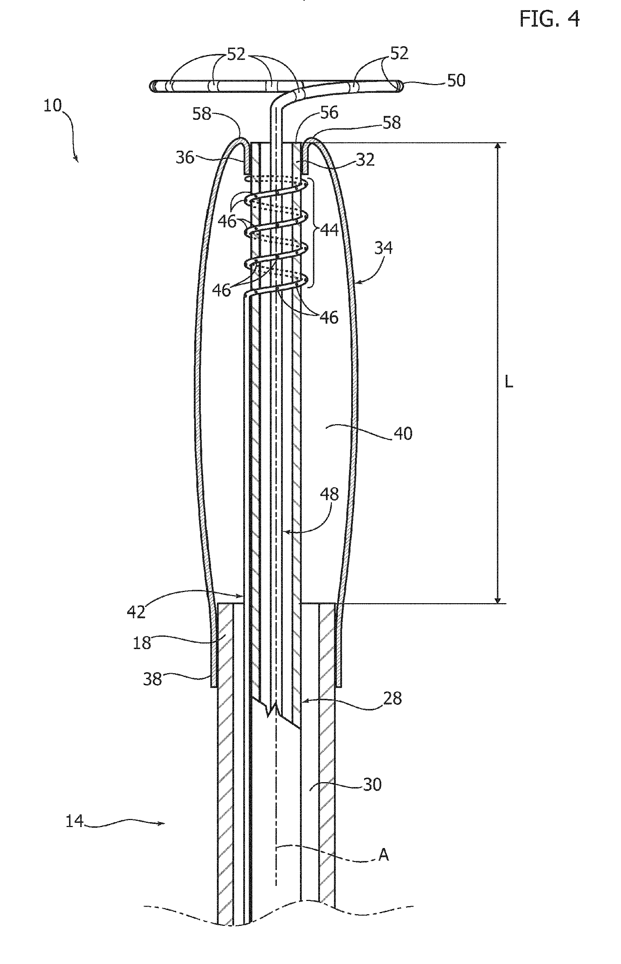

The mapping catheter lumen 28 is axially movable relative to the catheter body 14 between an insertion/extraction position illustrated in Figure 4 and an ablation position illustrated in Figures 2 and 3. In the insertion/extraction position, the distance L between the front ends of the mapping catheter lumen 28 and the catheter body 14 is greater than the corresponding distance L' in the ablation position.

In the insertion/extraction position illustrated in Figure 4, the cryogenic balloon 34 is deflated and is stretched out in an elongated shape. In the ablation position illustrated in Figures 2 and 3, the cryogenic balloon 34 is inflated by the cryogenic fluid injected into the cryogenic cavity 40. In the inflated position, the cryogenic balloon 34 has essentially a truncated ball shape, as shown in the figures, or a truncated-conical or "pear" shape, with the narrowest portion at the distal end 18 of the catheter body 14.

In the inflated position, the cryogenic balloon 34 has a front ablation area 58, which extends beyond the front surface 56 of the mapping catheter lumen 28. The front ablation area 58 is tangential to a plane perpendicular to the longitudinal axis A of the catheter body 14. The outer surface of the cryogenic balloon 34 has a circumferential ablation area 60, which surrounds the front ablation area 58. The circumferential ablation area 60 has a radius of curvature less than the radius of curvature of the front ablation area 58.

The end loop portion 50 of the mapping catheter 48 can be in the extracted position both with the cryogenic balloon 34 inflated (Figure 2) and with the cryogenic balloon 34 stretched out (Figure 4). With the cryogenic balloon 34 inflated, the end loop portion 50 of the mapping catheter 48 is facing the front ablation area 58.

With reference to Figure 4, when the cryogenic balloon 34 is in the stretched out position, the front ablation area 58 of the cryogenic balloon 34 forms a rounded toroidal tip around the distal end 32 of the mapping catheter lumen 28, with a front end that protrudes beyond the front surface 56 of the distal end 32 of the mapping catheter lumen 28.

When the cryogenic balloon 34 is inflated, the front ablation area 58 stretches and assumes an essentially flat position, which is useful for the cryogenic ablation of front portions of the tissues, such as the inner wall of the atrium.

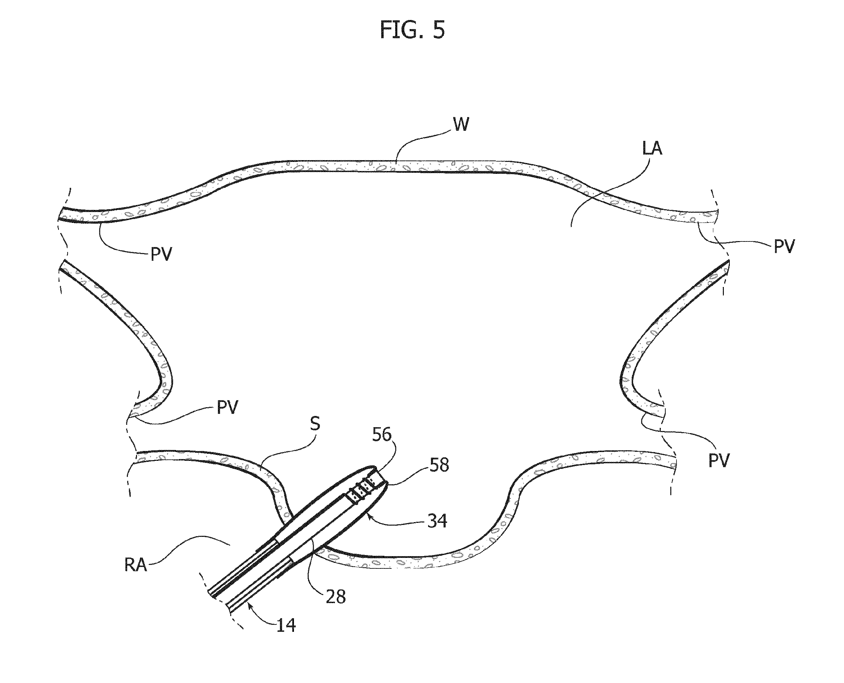

Figures 5-8 schematically illustrate the use of catheters according to the present invention for treating atrial fibrillation. As illustrated in Figure 5, the catheter according to the present invention, with the cryogenic balloon 34 stretched out, is inserted through the femoral vein until reaching the right atrium RA of the heart of the patient. Then, the catheter is inserted through a hole of the septum S that separates the right atrium RA from the left atrium LA. As already indicated previously, when the balloon catheter 34 is in the relaxed position, the front ablation area 58 has a rounded shape with a very small curvature radius.

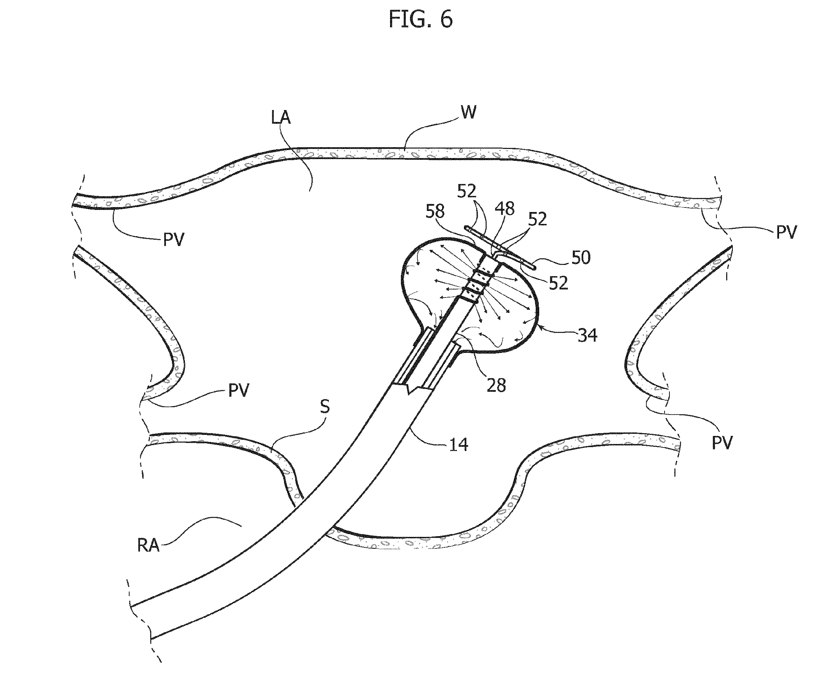

With reference to Figure 6, when the balloon catheter 34 is inserted within the left atrium LA, the cryogenic balloon 34 is inflated by injecting the cryogenic fluid into the cryogenic cavity 40. The loop portion 50 of the mapping catheter 48 is carried into the extracted position to carry out the mapping of the tissues to be treated.

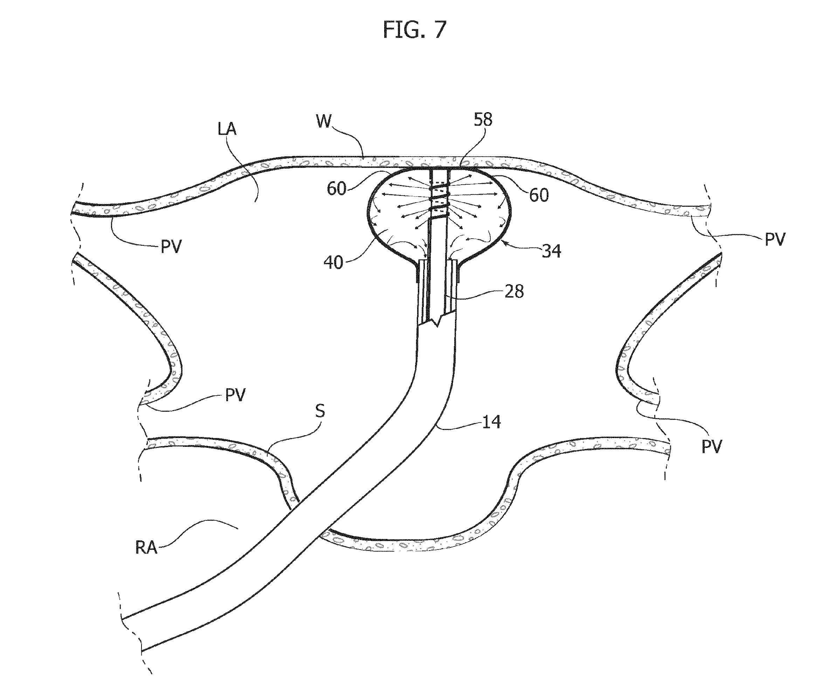

Figure 7 shows the step in which the cryogenic ablation of one wall W of the left atrium is carried out. After having identified the part of tissue to treat, the loop portion 50 of the mapping catheter 28 and the front ablation area 56 of the cryogenic balloon 34 is frontally placed alongside the wall portion W to be treated. A particularly relevant characteristic of the solution according to the present invention is that the front ablation area 56 establishes an extended contact with the wall W to be treated and blocks the blood flow to the area of tissue to be treated. In this way, the cryogenic energy does not become dispersed by the blood flow.

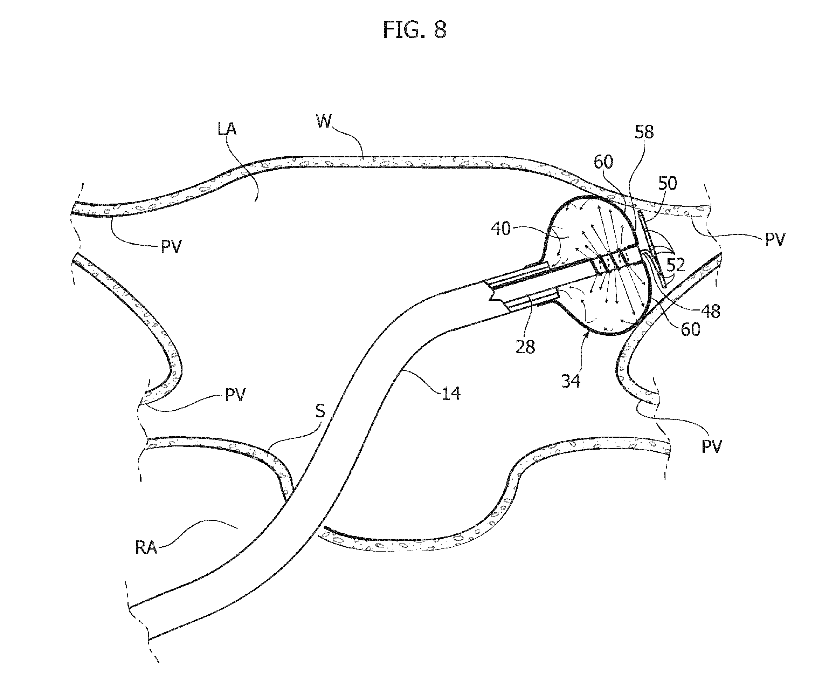

With reference to Figure 8, the cryogenic balloon 34 can also be used for the cryogenic ablation of the ostium of the pulmonary veins PV. In this case, the cryogenic ablation is made by the circumferential ablation area 60 of the cryogenic balloon 34. The circumferential ablation area 60 can be adapted to various diameters of the ostia of the pulmonary veins. The circumferential ablation portion 60 blocks the blood flow coming from the pulmonary veins during the cryogenic ablation. In this step, the end loop portion 50 of the mapping catheter 48 can be kept in the extracted position in order to identify the pulmonary vein PV and to obtain confirmation of the fact that the treatment has occurred in the correct way.

From the above description, it is evident that the same catheter can be used both to carry out ablation of the ostium of a pulmonary vein and to implement linear lesions on the free wall of the atrium. Therefore, the solution according to the present invention is particularly appreciated by cardiologists who wish to carry out treatment -by cryoablation- for paroxysmal, persistent or long-standing atrial fibrillation, minimizing the use of material and optimizing procedural times and vascular accesses.

## Claims

1. Catheter for cryogenic ablation, particularly for treating atrial fibrillation, comprising:
- a grip (12),
- a hollow catheter body (14) elongated along a longitudinal axis (A) and extending from said grip (12) up to a distal end (18),
- a mapping catheter lumen (28) extending within the catheter body (14) and axially movable relative to the catheter body (14), having a distal end (32) that extends beyond the distal end (18) of the catheter body (14),
- a tube for injecting cooling fluid (42), which extends between the mapping catheter lumen (28) and the catheter body (14) and having an injection zone (44) located between the distal end (18) of the catheter body (14) and the distal end (32) of the mapping catheter lumen (28),
- a mapping catheter (48) movable within the mapping catheter lumen (28) between an extracted position and a retracted position, wherein in the extracted position the mapping catheter (48) has an end loop portion (50) perpendicular to the longitudinal axis (A) of the catheter body (14) and in the retracted position said end loop portion (50) is extended and retracted within the mapping catheter lumen (28), and
- a cryogenic balloon (34) having a first end (36) fixed to the distal end of the mapping catheter lumen (28) and a second end (38) fixed to the distal end (18) of the catheter body (14), the cryogenic balloon (34) defining a cryogenic chamber (40) surrounding said injection zone (44) and expandable under the pressure of a cryogenic fluid injected from said injection zone (44), wherein said cryogenic balloon (34) assumes a stretched out insertion/extraction position and an inflated position for carrying out the cryogenic ablation treatments,
**characterized in that** the distal end (32) of said mapping catheter lumen (28) has an open and tip-free front surface (56), and **in that** said first end (56) of said cryogenic balloon (34) is folded-back towards the inside of said cryogenic chamber (40), so that in the inflated position, said cryogenic balloon (34) has a front ablation area (58) that extends beyond the front surface (56) of said mapping catheter lumen (28).

2. Catheter according to claim 1, **characterized in that** said first end (36) of the balloon catheter (34) has an outer surface that is directly fixed in contact with an outer surface of said distal end (32) of said mapping catheter lumen (28).

3. Catheter according to claim 1, **characterized in that**, in the inflated position, said cryogenic balloon (34) has essentially a truncated-ball or pear shape, with a narrower position at the distal end (18) of the catheter body (14).

4. Catheter according to claim 1, **characterized in that**, in the inflated position of the cryogenic balloon (34) said front ablation area (58) is tangential to a plane perpendicular to the longitudinal axis (A) of the catheter body (14).

5. Catheter according to claim 1, **characterized in that**, in the inflated position, said cryogenic balloon (34) has a circumferential ablation area (60), which surrounds said front ablation area (56).

6. Catheter according to claim 5, **characterized in that** said circumferential ablation area (60) has a curved profile with a radius of curvature less than the radius of curvature of said front ablation area (58).

7. Catheter according to claim 1, **characterized in that** said front ablation area (58), in the stretched out position, has the shape of a rounded toroidal tip that surrounds the distal end (32) of the mapping catheter lumen and which extends beyond the front surface (56) of the mapping catheter lumen (28).
